# EUROPEAN PATENT APPLICATION

(11) **EP 1 264 896 A1**
(43) Date of publication of application: **11.12.2002**
(21) Application number: 01202172.1
(22) Date of filing: 07.06.2001
(51) Int. Cl.: C12P 21/02, C07K 5/075

(54) **Enzymatic coupling of L-phenylalanine methyl ester and N-benzyloxycarbonyl-l-aspartic acid in a continuous or fed-batch process**

(71) Applicant: Holland Sweetener Company V.o.F., 6167 RA Geleen (NL)
(72) Inventor: Tokuda, Akira, Yamagata-shi, Yamagata 990-0829 (JP); Nakamura, Shiichiro, Shinnanyo-city, Yamaguchi (JP); Yamamoto, Norihiro, Shinnanyo-shi, Yamaguchi 746-00009 (JP); Quaedflieg, Peter Jan Leonard Mario, 6164 HB Geleen (NL)

(57) **Abstract**

The invention relates to a process for the preparation of N-benzyloxycarbonyl-α-L-aspartyl-L-phenylalanine methyl ester (Z-APM) by high-conversion enzymatic coupling of N-benzyloxycarbonyl-L-aspartic acid (Z-Asp) and L-phenylalanine methyl ester (L-PM) in a reaction mixture comprising an aqueous medium, a neutral protease as enzyme and an alkali metal salt, alkaline earth metal salt or ammonium salt, with formation of a precipitate. The enzymatic coupling of Z-Asp and L-PM is carried out in a continuous or fed-batch process at a pH of from 5.0 to 6.5 and an average charged molar ratio of Z-Asp and L-PM between 1:1 and 2:1 and wherein the actual molar ratio of Z-Asp and L-PM is higher than the average charge molar ratio. Preferably the alkali metal salt, alkaline earth metal salt or ammonium salt is present in an amount of from 3 to 25 %, calculated as % by weight based on the total reaction mixture.

## Description

The invention relates to a process for the preparation of N-benzyloxycarbonyl-α-L-aspartyl-L-phenylalanine methyl ester (Z-APM) by high-conversion enzymatic coupling of N-benzyloxycarbonyl-L-aspartic acid (Z-Asp) and L-phenylalanine methyl ester (L-PM) in a reaction mixture comprising an aqueous medium, a neutral protease as enzyme and an alkali metal salt, alkaline earth metal salt or ammonium salt, with formation of a precipitate.

N-protected α-L-aspartyl-L-phenylalanine methyl ester, such as, in particular Z-APM is an important precursor of the "intense sweetener" aspartame (hereinafter APM), a product having a sweetening power of approximately 200 times that of sucrose and with an excellent taste profile without, for example, the bitter aftertaste of other intense sweeteners such as, for example, saccharin and cyclamate. The sweetener aspartame is used, inter alia, in a wide range of products such as soft drinks, sweets, "table-top sweeteners", pharmaceuticals, etc.

Where mention is made in this application of Z-, this should also be understood to refer to any protective group related, in terms of apolar character, to Z-, such as, for example, benzyloxycarbonyl compounds substituted in the phenyl ring by one or more alkyl, alkoxy, acyl or halogen groups.

Among the methods known for the preparation of aspartame, enzymatic preparation methods owe their importance primarily to the fact that enzymatic coupling takes place in a stereoselective and regioselective manner. The enzymatic coupling reaction in question is an equilibrium-controlled reaction. High degrees of conversion in such enzymatic coupling reactions are, according to the state of the art, usually achieved when a precipitate is formed shifting the equilibrium to the synthesis side.

This equilibrium-control is demonstrated, for example, in US-A-4165311, which makes use of the fact that the equilibrium in the coupling reaction can be shifted to the right by the formation of a precipitating addition compound of N-protected aspartame, in particular of Z-APM, with D- or L-phenylalanine methyl ester present in the reaction mixture. Such addition compounds of the aspartame precursor are also designated by Z-APM·D-PM or Z-APM·L-PM, respectively.

This method, however, has a number of drawbacks. In order to form the addition compounds it is desirable, according to the state of the art, for the coupling reaction of Z-Asp and L-PM to be carried out with at least double the molar quantity of L-PM with respect to Z-Asp, or in the presence of an at least equivalent amount of D-PM. After the reaction the component(s) present in excess need to be recovered, resulting in extra handling and variable costs. A further disadvantage is that handling and further processing of the precipitated addition compound(s), in order to obtain the ultimately desired APM, is laborious.

The use of a large excess of L-PM or the use of an at least equivalent amount of D-PM is avoided in EP-A-664338, which describes an enzymatic coupling reaction in which from the start stoichiometric or virtually stoichiometric amounts of Z-Asp and L-PM·HCl are used, and in which also a precipitate is formed, which to a large extent consists of Z-APM. Said reaction is carried out, while stirring or shaking, under the influence of a neutral protease as an enzyme at an initial pH of from 4.5 to 6.0 and in the presence of an alkali metal salt, alkaline earth metal salt or an ammonium salt in a concentration in the range of from about 3 to 25 wt%, in a batch reactor or semi-continuously. If the process is carried out semi-continuously, only the formed precipitate is separated continuously, while further starting materials are being supplied in a virtually stoichiometric ratio and in a total amount equivalent in moles to the amount of precipitate separated.

A disadvantage of the process described in EP-A-664338 is the fact that, despite the use of stoichiometric or virtually stoichiometric amounts of Z-Asp and L-PM·HCl, next to Z-APM a large amount of the addition compound Z-APM·L-PM is initially formed under the conditions applied when the reaction is carried out at a relatively high pH, i.e. at a pH of for example 5.8 or higher, albeit to a lesser extent than in processes in which a large excess of L-PM is applied. The Z-APM·L-PM formed (co-)precipitates from the reaction mixture. As the formation of the addition compound Z-APM·L-PM requires two moles of L-PM for each mole of Z-Asp reacted, much more L-PM than Z-Asp is consumed so that the Z-Asp:L-PM molar ratio in solution is likely to become significantly higher than 1:1 during the course of the reaction. Such high ratios of Z-Asp:L-PM have been found to have a negative effect on the maximum conversion of Z-Asp and on the reaction rate.

Performing the reaction at a lower pH, for example at a pH of about 5, does to a certain extent avoid the formation of Z-APM·L-PM, but is in most cases not an attractive alternative as at such low pH the reaction rate will also be relatively low due to low enzyme activity and high enzyme deactivation. It is therefore difficult to perform the reaction under such conditions that a) Z-APM is formed immediately as (nearly) the sole precipitate and b) a reasonable reaction rate is obtained.

The object of the present invention is to provide a commercially attractive process for the enzymatic coupling of Z-Asp and L-PM, which affords high conversion of both Z-Asp and L-PM to directly obtain the Z-APM precipitate in combination with high enzyme activity and low enzyme deactivation, resulting in a favourable reaction rate.

Surprisingly, this object is achieved according to the invention by carrying out the enzymatic coupling of Z-Asp and L-PM in a continuous process or in a fed-batch process at a pH of from 5.0 to 6.5 and at an average charged molar ratio of Z-Asp and L-PM between 1:1 and 2:1 and wherein the actual molar ratio of Z-Asp and L-PM is higher than the average charged molar ratio.

Particularly surprising is the fact that, in contrast with the observations made for the batch process described in EP-A-664338, at average charged molar ratios of Z-Asp and L-PM as low as for example between 1.1:1 and 1.6:1, a precipitate consisting almost entirely of Z-APM is formed immediately and the formation of the Z-APM·L-PM addition compound (co)-precipitate is almost completely avoided, even when the coupling reaction is performed at a relatively high pH, for example at a pH higher than 5.8 but below 6.5. The possibility to conduct the coupling reaction at such high pH offers unique conditions to obtain high reaction rates and high conversion of the starting materials, while the direct formation of Z-APM precipitate saves the additional process steps which would have been required if Z-APM·L-PM had been formed.

Furthermore, it should be noted that carrying out the reaction continuously or in a fed-batch process according to the invention results in a surprisingly low enzyme deactivation.

In JP-A-97248197 a batch-wise process for the coupling of Z-Asp and L-PM is described using an excess of Z-Asp, i.e. at Z-Asp:L-PM molar ratios between 2:1 and 8:1, preferably of about 4:1. It is stated in the above patent application, that performing the reaction at an excess of Z-Asp offers the advantage of direct formation of Z-APM, while less L-PM needs to be recovered after the reaction and less L-PM is lost by hydrolysis during the reaction process.

However, although this embodiment indeed results in less L-PM consumption, instead the large excess of Z-Asp needs to be recovered in this case, thereby resulting in additional reaction steps with a high Z-Asp throughput. According to the present invention, in contrast, an average charged molar ratio of Z-Asp and L-PM lower than 2:1 is being used, and thus the quantity of the excess Z-Asp to be recovered is substantially reduced.

The term "average charged molar ratio of Z-Asp and L-PM" as used in the context of the present application, refers to the ratio between the amount of Z-Asp (in moles) totally charged during the course of the enzymatic coupling reaction and the amount of L-PM (in moles) totally charged during the course of the enzymatic coupling reaction.

The term "charged molar ratio of Z-Asp and L-PM" refers to the ratio between the amount of Z-Asp (in moles) charged and the amount of L-PM (in moles) charged at a certain time or during a certain part of the reaction period. The charged molar ratio may vary between wide limits, especially during the initial stages of the reaction, but will during the continuous feeding stage of a continuous (or fed-batch) process usually be in the range as indicated for the average charged molar ratio, as long as the average charged molar ratio, i.e. at the end of the process, is between 1:1 and 2:1.

The term "actual molar ratio of Z-Asp and L-PM" refers to the ratio between the amount of "free" Z-Asp (in moles) and the amount of "free" L-PM (in moles) present in the reaction mixture at a certain time or during a certain part of the reaction period.

As used in the present application, a continuous process is defined as a process in which at least one of the reactants is dosed continuously to the reactor and the reaction mixture is discharged continuously from the reactor. A continuous process as used in the present application may also comprise periods in which the enzymatic coupling reaction is carried out non-continuously, e.g. using a batch or fed-batch procedure, in which the reaction mixture is not discharged. The use of a batch and/or a fed batch procedure as part of the continuous process is particularly useful for starting up the reaction in the initial stages of the process and for completing the conversion at the end of the process. Applicant has found that for carrying out the continuous process according to the invention many forms are possible, both in terms of apparatus and in terms of the nature of the means which may be used to set or keep the system in motion. The process can be carried out in reactors, made from all kinds of materials such as glass, stainless steel, etc. which do not interfere with the enzymatic coupling reaction in a detrimental manner. The dimensions of the equipment may vary within wide limits. The reaction can therefore be carried out on any scale desired. In order to obtain a homogeneous reaction mixture, the actual Z-Asp and L-PM concentrations being approximately constant throughout the reaction mixture, the use of at least one well-mixed reactor is preferred. Examples of well-mixed reactors are a Continuously Stirred Tank Reactor (CSTR) and a column in which the reaction mixture is circulated. For economic reasons particularly one or two well-mixed reactors are applied. Using three or more well-mixed reactors could afford even higher conversions of both Z-Asp and L-PM, but would, on the other hand, result in higher investment costs and reduce the ease of operation. Most preferably two well-mixed reactors are applied in series, the advantage over one well-mixed reactor being that a lower Z-Asp to L-PM average charged molar ratio can be used with the same total residence time and conversion. When the reaction is carried out using at least one well-mixed reactor the process is preferably carried out in such a way that the product concentration is approximately constant over the well-mixed reactor and in time and approximately equals the product concentration of the discharged reaction mixture. A reactor with a plug flow behaviour like e.g. a column reactor may be used after the (series of) well-mixed reactor(s), for example in order to complete the conversion. If two or more reactors are applied, the reactors may have different reactor volumes.

As used in the present application, a fed-batch process is defined as a process in which at least one of the reactants is dosed to a batch reactor and in which the reaction mixture is not discharged continuously. Preferably at least one of the reactants is charged to the reactor before the start of the reaction in order to create a large excess of this reactant during the initial stages of the reaction. As meant herein, if more than one fed-batch reactors are being used, they are being operated in parallel. In a fed-batch process, in contrast to a continuous process, the amount (volume) of reaction mixture increases with time until the reaction is stopped. Applicant has found that for carrying out a fed-batch process according to the invention many forms are possible, both in terms of apparatus and in terms of the nature of the means which may be used to set or keep the system in motion. The process can be carried out in reactors, made from all kinds of materials such as glass, stainless steel, etc. which do not interfere with the reaction in a detrimental manner. The dimensions of the equipment may vary within wide limits. The reaction can therefore be carried out on any scale desired. Preferably the fed-batch reaction is carried out using a process with a high degree of back-mixing, more preferably in at least one stirred batch reactor.

In the scope of this application the term "continuous feeding stage" means the stage in which at least one of the reactants is dosed to the reaction mixture in time and can refer to both a continuous and a fed-batch process.

In the process according to the invention the actual molar ratio of Z-Asp and L-PM is higher than the average charged molar ratio. Incidentally, however, relatively short periods may occur, particularly at the end of the reaction, in which the actual molar ratio between Z-Asp and L-PM is lower than the average charged molar ratio, without significantly affecting the reaction rate and/or the product quality. Preferably, the actual molar ratio of Z-Asp and L-PM at the early stages of the reaction process, i.e. before reaching the continuous feeding stage, is for example at least 10 %, preferably at least 25 %, most preferably at least 40 % higher than the average charged molar ratio.

The feed rate of the reaction components may vary within wide limits. For economic reasons very low feed rates should be avoided, because such feed rates would result in a low output. On the other hand, extremely high feed rates, i.e. feed rates that go beyond the reactor capacity, should of course also be avoided. High feed rates should also be avoided because of potential inhomogeneity effects (so-called hot-spots) due to insufficient stirring capacity.

The agitation speed applied in the reactor(s) is not particularly critical and can easily be optimised by a person skilled in the art. Too high agitation speeds should be avoided, as such high agitation speeds might induce enzyme deactivation, particularly at relatively high slurry concentrations. On the other hand, extremely low agitation speeds should also be avoided, as such low agitation speeds could result in inhomogeneities in the reaction mixture, for example "pH hot spots", thereby also inducing enzyme deactivation.

The residence time in the case of a continuous reaction or the reaction time in the case of a fed-batch reaction is not particularly critical and may vary within a wide range. Optimisation can be performed by any person skilled in the art. In the case that more than one well-mixed reactor is applied in a continuous reaction, the residence time may be adjusted differently for each separate well-mixed reactor.

According to the present invention, the reaction is carried out using an average charged molar ratio of Z-Asp and L-PM between 1:1 and 2:1. Average charged molar ratios lower than 1:1 are likely to promote excessive formation of the Z-APM·L-PM adduct with the disadvantages mentioned above. At average charged molar ratios higher than 2:1 the excess of Z-Asp to be recovered from the reaction mixture after the coupling reaction will be unfavourably higher. Within the range between 1:1 and 2:1, an excess of Z-Asp leads to the best results in terms of degree of conversion and yield, while in the 1:1 situation, any required recycling of one or both of the unreacted starting materials is minimized. Preferably the reaction is carried out at an average charged molar ratio between 1.1:1 and 1.6:1.

In one embodiment of the invention, in which the reaction is carried out continuously using one CSTR, Z-Asp and L-PM are first reacted to Z-APM, for a sufficiently long time and at an initial actual molar ratio of Z-Asp and L-PM significantly higher than 2:1 in a batch or fed-batch process until reaching the continuous feeding stage. As soon as an appropriate level of conversion has been reached and the process can be stably converted into a continuous process, i.e. when Z-Asp and L-PM have first reached a degree of conversion to Z-APM of approximately 55 % or more, and the actual molar ratio between free Z-Asp and free L-PM is preferably still higher than 2:1, Z-Asp and L-PM are continuously added to the CSTR at a charged molar ratio between 1:1 and 2:1, and such feeding is continued until the average charged molar ratio of Z-Asp to L-PM is lower than 2:1, preferably lower than 1.6:1.

In another embodiment of the invention, in which more than one CSTR's are used (in series), the reaction is carried out similarly. The charged molar ratio of Z-Asp and L-PM is not necessarily equal for each of the subsequent CSTR's. When two CSTR's are used in series, preferably both Z-Asp and L-PM are charged to the first reactor using a relatively high [Z-Asp]/[L-PM] ratio. Preferably L-PM is charged to the second reactor together with the reaction mixture of reactor 1, the charged molar ratio of Z-Asp and L-PM to reactor 2 being such that the average charged molar ratio of Z-Asp to L-PM for the whole process is lower than 2:1, preferably lower than 1.6:1.

In yet another embodiment of the invention the reaction is carried out as a fed-batch reaction using one batch reactor. Z-Asp and L-PM are added to the batch reactor such that the average charged molar ratio is between 1:1 and 2:1 and the actual molar ratio of Z-Asp and L-PM is higher than 2:1 during at least the initial part of the process, preferably at least until a conversion of at least 55 % has been reached for the first time. The method of addition may be different for the different reactants, and may involve addition at once for part of the material at the start and/or during the course of the reaction, or addition in small portions during the course of the reaction, or by gradual dosing. Preferably, a large amount of Z-Asp is charged initially and L-PM is then added portionwise or dosed during the course of the reaction.

The pH during the reaction is between 5.0 and 6.5. Said pH limits can be passed shortly without an adverse effect on the results. It is preferable, however, for the pH during the coupling reaction to be held at a level below 6.5. More preferably, the pH is held below 6.2, most preferably below 6.0. At a pH below 5.0 the degree of conversion and the yields decrease due to lower enzyme activity. Preferably, a pH higher than 5.2 is applied, more preferably a pH higher than 5.5. In the case of a continuous process in which more than one well-mixed reactors are used, different pH values can be applied in the subsequent reactors. Incidentally, it should be noted that the lower limits of the pH may also be lowered to a limited extent, depending on the enzyme used. Thus, for example, when working with a mutant enzyme, which has optimum properties at a lower pH than the wild-type enzyme, a further lowering of the lower limit of the pH range as far as, for example, 3 to 5 will be achievable.

During the coupling reaction the pH of the reaction mixture may be adjusted using acids having a pKₐ between -10 and +8, preferably having a pKₐ between -2 and +7.5. Preferably acids are used which, under the applied reaction conditions, cause only little or no enzyme deactivation. Examples of such suitable acids are hydrochloric acid and acetic acid. Most preferably acetic acid is used. If necessary, a base may be added for adjusting the pH.

In another preferred embodiment of the process according to the invention, a buffer of an organic acid and a conjugated base thereof may be applied in the reaction mixture. Examples of suitable buffers are acetic acid/acetate, propionic acid/propionate, butyric acid/butyrate, citric acid/citrate, maleic acid/maleate and phthalic acid/phthalate. The advantage of using such buffers is that pH variations will be smaller while, during the course of the reaction process, the pH can still be adjusted with strong acids and/or bases, which in the absence of buffer would be more likely to cause enzyme deactivation. Preferably an acetic acid/acetate buffer is applied, the pH adjustment during the reaction being carried out using acetic acid or hydrochloric acid. When such buffers are used an increase of the solubility of Z-Asp, due to the presence of the buffer, should be avoided as much as possible, as a higher solubility results in less Z-Asp precipitate being recovered.

In yet another embodiment of the invention, the pH of the reaction mixture is adjusted by adjusting the pH of feed solutions containing raw materials, for example feed solutions containing Z-Asp, L-PM·HCl and/or enzyme, to the desired value.

In the process according to the invention use is made of an aqueous medium. The term aqueous medium, in the context of the present application, refers to any homogeneous, one-phase polar aqueous system, which may contain small amounts (up to approximately 30%) of an organic solvent such as, for example, methanol, acetonitrile, acetic acid, methyl-isobutyl ketone (MIBK) and toluene.

According to the invention the aqueous medium contains some alkali metal salt, alkaline earth metal salt or ammonium salt, preferably in an amount of from 3 to 25%, calculated as % by weight based on the total reaction mixture. Various alkali metal salts, alkaline earth metal salts or ammonium salts can be used in the process according to the invention. Suitable examples are halides or sulphates of potassium, sodium, lithium, and ammonium, or mixtures thereof. The term ammonium here also refers to ammonium substituted with one or more C₁₋₃ alkyl groups; examples of such substituted ammonium salts are tri(m)ethyl ammonium chloride, di(m)ethyl ammonium chloride, etc. As far as the percentage by weight range is concerned, which according to the invention is preferably in the range from 3 to 25 wt%, the potential applications are partially determined by the solubility of the respective salts. Alkali metal and ammonium salts generally have the best solubility and are to be preferred. Particular preference is given to use of lithium chloride, sodium chloride, potassium chloride, sodium sulphate, potassium sulphate, ammonium chloride and/or ammonium sulphate. Most preferably sodium chloride is used.

The higher the salt content in the reaction system, the faster the conversion proceeds, without the yields being affected significantly. At higher contents, however, the viscosity of the system will soon increase strongly and/or the solubility limit of one or more of the starting materials and/or of the salt itself will be exceeded, so that the precipitate obtained is unnecessarily contaminated with such starting material or salt, so that the degree of conversion of the reaction is lower. Above 25 wt%, the viscosity of the system makes it virtually impossible to carry out the reaction. The lower the salt content in the reaction system, the longer the total reaction time required will be, giving rise to increased hydrolysis of, in particular, L-PM. At lower salt concentrations, e.g. below 3 wt%, there also is an undesirable effect on the solubility of the coupling product. If the addition product (Z-APM·L-PM) should precipitate prematurely, this, incidentally, does not interfere in the reaction according to the invention since the shift in equilibrium will automatically result in conversion of all or part of this product into Z-APM precipitate during the course of the reaction under the specific conditions in question. Preferably, the salt content is from 10 to 18 wt%, because in that range the most favourable conditions are found with respect to a combination of a) the viscosity of the system; b) the solubility of the starting materials and precipitate formation of the end product; c) the reaction time and d) the purity of the Z-APM precipitate.

The enzymatic coupling usually takes place within a temperature range of from 10 to 60°C. The lower the temperature, the lower the rate at which both the coupling reaction and the side reactions, such as hydrolysis of L-PM and Z-APM, proceed. The higher the temperature, the faster deactivation of the enzyme will occur. Those skilled in the art can readily determine what temperature should be chosen for the enzyme used in order to achieve optimum results in terms of conversion to Z-APM and stability of the enzyme.

The enzymatic coupling according to the invention is carried out using a neutral protease. The term neutral protease here refers to any known neutral proteolytic enzyme which can be used in the synthesis of Z-APM from Z-Asp and L-PM, as well as mutants thereof having a comparable or even increased activity. Examples include metallo-proteases such as thermolysin, produced by *Bacillus thermoproteolyticus*, and other proteases produced, inter alia, by various *Bacillus* species, such as *Bacillus stearothermophilus, Bacillus amyloliquefaciens, Bacillus cereus,* collagenase, etc. In general, these enzymes exhibit an optimum in protease activity at a pH of from approximately 6 to 8, but it has been found that, when they are used under the conditions according to the present invention, good results are also achieved at the initial pH of from 5.0 to 6.5, in particular from 5.2 to 6.2, more in particular from 5.5 to 6.0, without the need for employing excessive additional amounts of enzyme. It should be noted that the presence of small amounts of Ca²⁺ ions in general has a beneficial effect on the stability and the activity of the enzyme.

In the continuous or fed-batch process according to the invention, the activity of the enzyme is, generally, not changed or hardly changed, which permits the reaction to be run for a long time with recycling of the enzyme. Consequently, it is recommended - in particular when using dissolved enzymes - for the enzyme to be re-employed for the enzymatic coupling reaction, after separation from the precipitate obtained.

The amount of enzyme used in the coupling reaction is not very critical, but usually such an amount of enzyme will be used that a high degree of conversion is reached within an acceptable, limited period of time. Generally, amounts of enzyme (which is herein understood to be the protein having the enzyme activity in question, the so-called active protein) of from 0.08 to 1.5%, preferably from 0.15 to 0.75%, expressed as per cent by weight based on the total reaction mixture, are suitable. The percentages mentioned here generally correspond to from approximately 0.5 to 10%, or preferably from 1 to 5%, if the amount of enzyme is given as the total amount of (dry) enzyme preparation employed, i.e. active protein and other proteins as well as other adjuvants, such as salts. The enzymes will often be employed as an enzyme preparation and are also commercially available as such. Usually, the amount of active protein in such a preparation is approximately 15% of the weight of the preparation.

In the process according to the invention the enzyme can be used in any form suitable for this purpose, i.e. both in dissolved and in immobilized form. If an immobilised enzyme catalyst is used, the particle size and/or density of the immobilised enzyme particles preferably deviates significantly from the particle size and density of the Z-APM precipitate in order to facilitate separation of the precipitate while keeping the enzyme in the reaction medium. Preferably, use is made of dissolved enzyme, obtained by dissolving an enzyme preparation in the reaction medium, as this has advantages in the separation of the Z-APM precipitate obtained and further processing thereof, as well as in the reuse of the biocatalyst itself. As stated earlier, it is also possible to use mutants of the enzymes in question. The percentages specified herein-above for the amount of enzyme can vary, depending on the activity of the enzyme to be used, and will certainly vary when mutant enzymes are used.

Where reference is made in this application to L-phenylalanine methyl ester (L-PM), this should also be understood to refer to the acid salts derived therefrom, such as, for example, the hydrochloride salt (L-PM·HCl). Where reference is made in this application to benzyloxycarbonyl aspartic acid (Z-Asp), this should also be understood as referring to the salts derived therefrom, such as, for example, the disodium salt (Z-Asp·diNa). Obviously, when using an acid salt instead of L-PM and/or a salt instead of Z-Asp, it will be necessary, to a limited extent, to adjust the amounts of chemicals to be employed to achieve the desired pH.

The concentrations of the starting materials Z-Asp and L-PM may vary within wide limits and are determined, inter alia, by the solubility of these materials in the reaction mixture. However, the presence of small amounts of undissolved starting materials does not interfere with the course of the reaction as long as the continuous feeding stage has not been reached.

In a preferred embodiment of the invention the non-converted Z-Asp is recovered. After leaving the coupling reactor(s) and optionally after removal of the enzyme by, for example, ultrafiltration, the reaction mixture may subsequently be subjected to an extraction process at low pH, i.e. at a pH lower than for example 4, using an a water-immiscible organic solvent, for example an ester, a ketone, an optionally substituted aliphatic or aromatic hydrocarbon, preferably toluene or MIBK, as the extraction solvent in order to extract at least a part of the Z-Asp from the reaction mixture into the organic solvent as the free diacid. Later, the Z-Asp may be back-extracted as its salt from the organic phase using a sufficient amount of base. The aqueous solution containing the Z-Asp salt can then be recycled to the reaction process. The processes of extraction and back-extraction can be optimised by any person skilled in the art.

In a preferred embodiment of the invention, a small amount of organic solvent is added to the reaction mixture during the course of the enzymatic coupling reaction. It has surprisingly been found that the presence of such organic solvents significantly decreases the rate of enzyme deactivation. Organic solvents which are suitable for this purpose are water-immiscible solvents, for example toluene, MIBK and mixtures thereof. Preferably the same organic solvent is used that is also used for the recovery of Z-Asp. Toluene is most preferred, because it is being liberated from the Z group during hydrogenolysis and as such is not a process-strange compound.

The invention will now be explained in more detail with reference to the following examples and the comparative example, without however being limited thereto.

### Experimental procedures

As the enzyme a commercially available wild type thermolysin enzyme (from Amano) was used in all cases. The enzyme concentrations and initial enzyme activities specified in the following examples are in each case calculated on the basis of the amount of enzyme preparation employed.

The degrees of conversion of Z-Asp and L-PM mentioned in the examples 1-4 were determined by means of gel filtration high performance liquid chromatography (gel filtration HPLC), using UV-spectrophotometric detection at 254 nm, applying a column charged with TSK-Gel G2000SW and acetonitrile/water 70/30 (v/v) with 0.15 M acetate buffer as the eluent at pH 5.6 and at T = 25 °C.

The amount of enzyme was analysed by reversed-phase HPLC using UV-spectrophotometric detection at 280 nm, applying a column charged with TSK-Gel Phenyl-5PW RP. As the eluent a gradient was used of A: acetonitrile/water 5/95 (v/v) with 0.15 M calcium acetate buffer at pH 6 and B: acetonitrile/water 60/40 (v/v) with 0.15 M calcium acetate buffer at pH 6. Gradient: 0-4 min: A/B = 90/10 constant; 4-10 min : A/B = 90/10 to 40/60 linearly in 6 min.

The samples were taken from the reaction mixture in such a way, that this did not significantly influence the outcome of a reaction. In the case of Comparative example A they were immediately taken up in methanol in order to stop the enzymatic reaction and were stored at low temperature prior to being analysed (via auto-injection into the continuous stream of the eluent). In the case of examples 1-4 the samples were diluted with the eluent used for HPLC.

In all of the examples initial reaction mixtures are described, including their concentrations [Z-Asp], [L-PM], [Ca²⁺], [NaCl] and [enzyme]. The mixtures were prepared as follows, unless noted otherwise:
- solid Z-Asp and NaCl were dissolved in aqueous NaOH ("Z-Asp.Na solution", NaOH:Z-Asp = 1.7 mol/mol).
- solid L-PM.HCl was dissolved in a small amount of water ("L-PM.HCl solution" with pH of ca. 3.5)
- the Z-Asp.Na was added slowly, under stirring, to the L-PM.HCl solution and the pH was roughly adjusted to the desired value using an aqueous NaOH solution
- to this mixture an aqueous solution of the enzyme and CaCl₂ was added
- finally, the pH was adjusted to its final (initial) value using aqueous NaOH.

### Comparative example A

### Batch reaction

A solution was prepared containing:
[Z-Asp] = 0.74 mol/kg
[L-PM] = 0.65 mol/kg
[enzyme] = 3.8 wt%
[NaCl] = 13.0 wt%
pH = 5.3.

The solution was stirred (75 rpm) at 40 °C and the pH was adjusted to and kept at 5.3 during the whole coupling reaction using a 16 wt% aqueous HCl solution. During approximately 5 hours the conversion of L-PM vs. reaction time increased linearly with 12.0 %/h (as the first order coefficient), corresponding with 40% conversion of L-PM after 5 h. After the formation of a precipitate, samples were taken at several time intervals and analysed. In all samples analysed the solid appeared to be mainly Z-APM·L-PM. Between 5 h and 7 h reaction time the conversion increase curved down and from 7 h (corresponding to about 45% conversion of L-PM) to 30 h (corresponding with 84% conversion of L-PM) the conversion vs. time was linear again with 1.8 %/h as the first order coefficient. From 30 h to 44 h the conversion increase slowed down further to give 96% conversion of L-PM after 44 h.

Comparative example A shows that Z-APM·L-PM is formed in the early stages of the reaction. Because of the relatively high consumption of L-PM for the formation of Z-APM·L-PM, a relatively low concentration of "free" L-PM is left after 5 h, causing the reaction to slow down. After ca. 7 h a very small amount of "free" L-PM is left and the enzymatic reaction rate is determined by the rate of liberation of L-PM from Z-APM·L-PM. Applicants have similar experiences with reactions performed at Z-Asp:L-PM ratios of up to 2:1.

### Example 1

### Continuous reaction in one CSTR, pH = 5.2

In this example one CSTR with a volume of 5 liter was used. The reaction was started as a batch reaction, subsequently performed as a fed-batch reaction (L-PM solution was added) and finally as a continuous reaction by adding both a Z-Asp/enzyme solution and an L-PM solution.

### Batch-wise reaction: (from 0 - 17 h)

A solution with the following contents was prepared, in which the last step was the addition of the enzyme:
[Z-Asp] = 0.53 mol/kg
[L-PM] = 0.27 mol/kg
[enzyme] = 4.2 wt%
[NaCl] = 13 wt%
[Ca²⁺] = 8 mmol/kg

The mixture (total weight 4.0 kg) was transferred to the reactor and subsequently stirred (agitation speed: 280 rpm) at 40 °C for 17 h. During this period, the pH was carefully adjusted to 5.2 with 20 wt% aqueous HCl. Several samples were taken, filtered and washed with a small portion of water. It appeared that in all cases the ratio Z-APM : Z-APM·L-PM in the solid was > 99:1.

### Fed-batch reaction (from 17 - 22 h)

To the mixture obtained above was added, with a feed rate of 69 g/h a solution containing:
[L-PM] = 2.0 mol/kg
[Ca²⁺] = 30 mmol/kg
at a temperature of 40 °C and with continuous pH adjustment to 5.2 using 20 wt% aqueous HCl.

At t = 22 h, i.e. directly after the fed-batch reaction, the conversions of Z-Asp and L-PM had reached 66% and 90%, respectively.

### Continuous reaction (from 22 - 42 h)

To the mixture obtained above were added continuously:
· a solution, containing
   [Z-Asp] = 0.61 mol/kg
   [enzyme] = 5 wt%
   [NaCl] = 10 wt%
   with a feed rate of 193.4 g/h
   and
· a solution, containing
   [L-PM] = 2.0 mol/kg
   [Ca²⁺] = 30 mmol/kg
   with a feed rate of 44.6 g/h

The reaction mixture was stirred at 40 °C and the pH adjusted to 5.2 with 20 wt% aqueous HCl. The residence time was 19 h and the charged molar ratio Z-Asp/L-PM was 1.33 mol/mol. From 22 h to 42 h the Z-Asp and L-PM conversion levels were stable (65% and 90%, respectively) and also the amount of enzyme remaining was constant (80%). On several time intervals samples were taken and filtered; in the solid the ratio [Z-APM] : [Z-APM·L-PM] was > 98 : 2. The stable output of Z-APM was 34 g/h (which is a yield of 89 % based on L-PM).

### Example 2

### Fed-batch reaction at high pH = 5.8, [NaCl] 13 wt%

A starting solution was prepared, containing the following components:
[Z-Asp] = 0.734 mol/kg
[L-PM] = 0.125 mol/kg
[NaCl] = 12 wt%
[Enzyme] = 3.4 wt%
[Ca²⁺] = 7.8 mmol/kg
Total weight: 2.50 kg
pH = 5.8

The mixture was stirred at 40 °C for 49 h, with the pH continuously being adjusted to 5.8 with 20 wt% aqueous HCl. During this time an L-PM feed solution ([L-PM] = 2.0 mol/kg) was added, with a feed rate of 12.46 g/h. The average charged molar ratio between Z-Asp and L-PM was 1.17 mol/mol.

After 49 h, when all L-PM·HCl feed solution had been added, the conversions of Z-Asp and L-PM were 81.2% and 96.1%, respectively. The enzyme remaining was approx. 49%. This slurry was used for the continuous experiment in Example 3 (reactor 2). From the analysis of samples taken from the coupling slurry which were filtered and washed with water, it appeared that the precipitate was (almost) solely Z-APM.

### Example 3

### Continuous reaction in 2 CSTR's, pH = 5.8

In this example two CSTR's with a volume of 5 liter were used.

### Fed-batch reaction in reactor 1

A mixture was prepared, containing the following components:
[Z-Asp] = 0.75 mol/kg
[L-PM] = 0.125 mol/kg
[NaCl] = 11 wt%
[Enzyme] = 3.5 wt%
[Ca²⁺] = 10 mmol/kg
Total weight: 3.49 kg

The mixture was stirred at 40 °C for 24 h. During this time a L-PM·HCl feed solution ([PM] = 2.0 mol/kg) was added, with a feed rate of 29.79 g/h. The pH was controlled at 5.8 using 20 wt% aqueous HCl. The Z-Asp and L-PM conversions were 69% and 95%, respectively and the enzyme remaining was 94 %. The volume of this slurry was 3.5 liter corresponding to 4.2 kg.

### Continuous reaction in reactor 1

To this slurry were added, simultaneously, the following two solutions:
- A Z-Asp/enzyme solution (the pH of which was adjusted to 6.5), with a feed rate of 163.4 g/h, containing [Z-Asp] = 0.80 mol/kg, [enzyme] = 3.5 wt% and [Ca²⁺] = 10 mmol/kg
- A L-PM·HCl solution, with a feed rate of 46.6 g/h, contained [L-PM] = 2.0 mol/kg.

The moment of the simultaneous start of the addition of these solutions to the slurry is called t = 0. The mixture was stirred at 40 °C and the pH was adjusted to 5.8 with 20 wt% aqueous HCl. The residence time was 20 h. The product stream was 219 g/h, which was collected until t = 24 h. The reaction was monitored; it was observed that the continuous operation was very stable. The Z-Asp and L-PM conversions were constantly 68% and 95% respectively.

### Continuous reaction in reactor 1 and reactor 2

At t = 24 h, a part of the slurry (2.6 kg) obtained in Example 2 was charged into reactor 2 to be used as initial slurry for the start-up of reactor 2. The reactor was closed at the bottom, so nothing was discharged at that moment.

### Reactor 1

From t = 24, reactor 1 was maintained in the stable state, with that difference, that the slurry coming out of reactor 1 was not collected but charged into reactor 2. All the time, the reactors and all solutions were kept at 40 °C and the pH was adjusted to 5.8 using 20 wt% aqueous HCl. The amounts of starting materials charged in reactor 1 were as follows: [Z-Asp] = 0.63 mol/kg and [L-PM] = 0.45 mol/kg, the average charged molar ratio **for reactor 1** being 1.4 : 1. The enzyme concentration was 2.7 wt% . The total weight of the reaction mixture was 4.2 kg.

The residence time in reactor 1 was 20 h.

### Reactor 2

Besides what already was present in the reactor and the continuous flow coming from reactor 1 (see above) also an additional L-PM·HCl solution was added, having [L-PM] = 2.0 mol/kg with a feed rate of 7.7 g/h. The continuous discharge of slurry from reactor 2 was started when the total weight of the slurry in reactor 2 amounted to 4.35 kg (corresponding with a volume of 3.6 liter). In all stages the temperature was kept at 40 °C and the pH was adjusted to 5.8 using 20 wt% aqueous HCl. The amounts of starting materials charged in reactor 2 were as follows: [Z-Asp] = 0.60 mol/kg and [L-PM] = 0.50 mol/kg, the average charged molar ratio being 1.2 : 1 **for reactor 2**. The enzyme concentration was 2.6 wt%. The residence time in reactor 2 was 20 h.

The concentrations of all components were very stable during the whole experiment (which was run until t = 100 h). The Z-Asp and L-PM conversions were constant (82% and 98% respectively) and the enzyme remaining in reactor 2 was 55% - 60%.

This example shows that a 1:1 continuous coupling in 2 CSTR's is very well feasible at pH = 5.8. However, reaction conditions can easily be optimized by a person skilled in the art in order to maximize the amount of active enzyme remaining, see for example Example 4.

### Example 4

### Continuous reaction in 2 CSTR's, pH = 5.8

In this example two CSTR's with a volume of 5 liter were used.

### Fed-batch reaction in reactor 1

A mixture was prepared, containing the following components:
[Z-Asp] = 0.75 mol/kg
[L-PM] = 0.13 mol/kg
[NaCl] = 13.0 wt%
[Enzyme] = 3.5 wt%
[Ca²⁺] = 10 mmol/kg
Total weight: 3.5 kg.

The mixture was stirred (180 rpm) at 40 °C for 48 h. During this time a L-PM·HCl feed solution ([L-PM] = 2.0 mol/kg) was added, with a feed rate of 15.0 g/h. The pH was controlled at 5.8 using an aqueous 50 wt% AcOH solution. The amounts of starting materials charged in reactor 1 were as follows: [Z-Asp] = 0.623 mol/kg and [L-PM] = 0.443 mol/kg, the charged molar ratio being 1.4:1. Z-Asp and L-PM conversions were 70% and 95%, respectively, and a remaining enzyme yield was 91%. In order to convert this mixture into the initial mixture for the continuous coupling in reactor 1, the amount of enzyme was completed again to 100% remaining.

### Continuous reaction in reactor 1

To the above slurry were simultaneously added the following 3 solutions. The moment of the simultaneous start of the addition of these solutions is called t = 0 h.
1. A Z-Asp/enzyme solution, added at a feed rate of 163.4 g/h, which contained:
   [Z-Asp] = 0.80 mol/kg
   [NaCl] = 14.5 wt%
   [enzyme] = 3.5 wt%
   [Ca²⁺] = 10 mmol/kg
   pH = 6.0
2. A L-PM·HCl solution, added at a feed rate of 46.6 g/h, contained [L-PM] = 2.0 mol/kg
3. A small amount of aqueous 50 wt% acetic acid to adjust the pH.

The charged molar ratio of Z-Asp/L-PM is 1.4 mol/mol. At t = 0, also the discharge from reactor 1 to reactor 2 (which was still empty) started, with a feed rate of 210 g/h. The reactor volume was 3.5 liter (4.2 kg). The residence time was 20 h. The mixture was stirred (with a rate of 180 rpm) at 40 °C and the pH was adjusted to 5.8 with aqueous 50 wt% acetic acid.

The reaction was monitored by taking samples and analyzing [Z-Asp], [L-PM], [Z-APM], [L-Phe] etc.; it was observed that the continuous operation was very stable for 100 h. The Z-Asp and L-PM conversions were almost constantly 66.5% and 95.2% respectively, and the enzyme remaining was constantly 88.5%.

### Continuous reaction in reactor 2

From t = 0, the slurry of 1st reactor was discharged to 2nd reactor. At t = 22.5 hours, the slurry in 2nd reactor was started to discharge continually. From t = 22 h the following solutions were added to reactor 2:
1 an L-PM solution (with [L-PM] = 2 mol/kg), added with a feed rate of 7.7 g/h
2 toluene with a feed rate of 24.3 g/h
3 a small amount of aqueous 50 wt% acetic acid to adjust the pH.

The charged molar ratio [Z-Asp]/[L-PM] was 1.2 mol/mol. The mixture was stirred (180 rpm) at 40 °C and the pH was adjusted to 5.8 with aqueous 50 wt% acetic acid. The residence time was approximately 20h. The reaction was monitored by taking samples and analyzing [Z-Asp], [L-PM], [Z-APM], [L-Phe] etc.; it was observed that the continuous operation was very stable for 100 h. The Z-Asp and L-PM conversions were almost constantly 78.5% and 97.7% respectively, and the enzyme remaining was constantly 88.0%.

## Claims

1. Process for the preparation of N-benzyloxycarbonyl-α-L-aspartyl-L-phenylalanine methyl ester (Z-APM) by high-conversion enzymatic coupling of N-benzyloxycarbonyl-L-aspartic acid (Z-Asp) and L-phenylalanine methyl ester (L-PM) in a reaction mixture comprising an aqueous medium, a neutral protease as enzyme and an alkali metal salt, alkaline earth metal salt or ammonium salt, with formation of a precipitate, **characterized in that** the enzymatic coupling of Z-Asp and L-PM is carried out in a continuous or fed-batch process at a pH of from 5.0 to 6.5 and an average charged molar ratio of Z-Asp and L-PM between 1:1 and 2:1 and wherein the actual molar ratio of Z-Asp and L-PM is higher than the average charge molar ratio.

2. Process according to Claim 1, **characterized in that** the enzymatic coupling is carried out in a continuous process using at least one well-mixed reactor.

3. Process according to Claim 2, **characterized in that** two well-mixed reactors are used in series.

4. Process according to any one of Claims 1-3, **characterized in that** the average charged molar ratio of Z-Asp and L-PM is between 1.1:1 and 1.6:1.

5. Process according to any one of Claims 1-4, **characterized in that** the pH is in the range of from 5.5 to 6.0.

6. Process according to any one of Claims 1-5, **characterized in that** during the coupling reaction the pH of the reaction mixture is adjusted using an acid, preferably acetic acid.

7. Process according to any one of Claims 1-6, **characterized in that** the reaction mixture contains a buffer.

8. Process according to claim 7, **characterized in that** the buffer is chosen from the group consisting of acetic acid/acetate, propionic acid/propionate, butyric acid/butyrate, citric acid/citrate, maleic acid/maleate and phthalic acid/phthalate buffers.

9. Process according to any one of Claims 1-8, **characterized in that** the alkali metal salt, alkaline earth metal salt or ammonium salt is present in an amount of from 3 to 25 %, calculated as % by weight based on the total reaction mixture.

10. Process according to any one of Claims 1-9, **characterized in that** the enzymatic coupling takes place at a temperature between 10 and 60 °C.

11. Process according to any one of Claims 1-10, **characterized in that** the enzymatic coupling is carried out in the presence of from 0.08 to 1.5 wt% of enzyme (active protein), based on the total reaction mixture.

12. Process according to any one of Claims 1-11, **characterized in that** a water immiscible organic solvent is added to the reaction mixture during the course of the coupling reaction.

13. Process according to Claim 12, **characterised in that** the water immiscible organic solvent is toluene or methyl-isobutyl ketone (MIBK).

14. Process according to any one of Claims 1-13, **characterized in that** the actual molar ratio of Z-Asp and L-PM before reaching the continuous feeding stage is at least 40 % higher than the average charged molar ratio.

15. Process according to any one of Claims 1-13, **characterized in that** after the coupling reaction the reaction mixture is filtrated to yield solid Z-APM and a mother liquor, the mother liquor is subjected to an extraction process at a pH < 4 using an organic solvent, in which at least part of the Z-Asp is extracted from the mother liquor into the organic solvent, followed by back-extraction of the Z-Asp as a Z-Asp salt from the organic solvent into an aqueous solution at pH > 4.5.
